# EUROPEAN PATENT SPECIFICATION

(11) **EP 4 436 589 B1**
(45) Date of publication and mention of the grant of the patent: **17.06.2026**
(21) Application number: 23707799.5
(22) Date of filing: 03.02.2023
(51) Int. Cl.: A61K 35/745, A61K 35/747, A61P 11/06, A61K 35/00

(54) **PROBIOTIC COMPOSITIONS AND THEIR USE IN MODULATING ALLERGY-TYPE INFLAMMATORY ACTIVITY**
PROBIOTISCHE ZUSAMMENSETZUNGEN UND IHRE VERWENDUNG ZUR MODULATION DER ENTZÜNDUNGSAKTIVITÄT VOM ALLERGIETYP
COMPOSITIONS PROBIOTIQUES ET LEUR UTILISATION DANS LA MODULATION DE L'ACTIVITÉ INFLAMMATOIRE DE TYPE ALLERGIE

(30) Priority: 03.02.2022 IT 202200001859
(43) Date of publication of application: 02.10.2024
(73) Proprietor: SYNBALANCE SRL, 21040 Origgio (VA) (IT)
(72) Inventor: MALANCHIN, Rosella, 21040 Origgio (VA) (IT); PIANGIOLINO, Cristiana, 21040 Origgio (VA) (IT); CASTEGNARO, Silvia, 21040 Origgio (VA) (IT); MALFA, Patrizia, 21040 Origgio (VA) (IT)
(74) Representative: Benedetto, Marco
(86) International application number: PCT/IB2023/050991
(87) International publication number: WO 2023/148681

(56) References cited:
- WO-A1-2021/024216
- IT-A1- MI20 121 173
- ANONYMOUS: "Advanced Pro-Veflora 50 Billion | Massage Schmidmair", 28 January 2021 (2021-01-28), XP055887890, Retrieved from the Internet <URL:http://web.archive.org/web/20210128090812/https://www.schmidmair.at/produkt/nahrungsergaenzungen/advanced-pro-veflora-50-billion> [retrieved on 20220207]
- I. PRESTI ET AL: "Evaluation of the probiotic properties of new Lactobacillus and Bifidobacterium strains and their in vitro effect", APPLIED MICROBIOLOGY AND BIOTECHNOLOGY, vol. 99, no. 13, 7 March 2015 (2015-03-07), pages 5613 - 5626, XP055200124, ISSN: 0175-7598, DOI: 10.1007/s00253-015-6482-8
- CICERO ARRIGO F G ET AL: "Impact of a short-term synbiotic supplementation on metabolic syndrome and systemic inflammation in elderly patients: a randomized placebo-controlled clinical trial", EUROPEAN JOURNAL OF NUTRITION, vol. 60, no. 2, 16 May 2020 (2020-05-16), pages 655 - 663, XP037376916, ISSN: 1436-6207, DOI: 10.1007/S00394-020-02271-8
- MICHELOTTI ANGELA ET AL: "Efficacy of a probiotic supplement in patients with atopic dermatitis: a randomized, double-blind, placebo-controlled clinical trial", EUROPEAN JOURNAL OF DERMATOLOGY., vol. 31, no. 2, 1 April 2021 (2021-04-01), FR, pages 225 - 232, XP055886899, ISSN: 1167-1122, DOI: 10.1684/ejd.2021.4019

## Description

### TECHNICAL FIELD OF THE INVENTION

The present invention relates to probiotic compositions comprising one or more strains belonging to the genus *Lactobacillus* and one or more strains belonging to the genus *Bifidobacterium* that are useful in modulating allergy-type inflammatory activity and, therefore, are compositions useful in the prevention and/or treatment of allergies, respiratory allergies, preferably seasonal allergies such as hay fever (hay fever denotes those forms of allergic rhinitis that recur annually and is a reaction of the mucosa of the eyes, nose and airways to seasonal pollens, but also to dust, certain types of volatile substances, animal hair (especially feline) and other irritants), allergic rhinitis (allergic rhinitis is an inflammation of the inner part of the nose caused by a substance, called an allergen, such as pollen, dust, mold or animal hair. Allergic rhinitis is a very common condition that causes cold-like disorders (symptoms) such as sneezing, itching, stuffy nose and mucus production), allergic rhinoconjunctivitis (allergic rhinoconjunctivitis is an inflammatory process of the nasal mucosa due to an immunological reaction induced by allergens, usually inhalants, to which the atopic patient is specifically sensitized) and allergic asthma (allergic asthma is an inflammation of the bronchial tree, caused by exposure to allergens habitually dispersed in the environment and harmless to healthy individuals; among possible allergens, the most common are pollen, pet dander and hair, dust mites and mold) or allergies related to *Staphylococcus aureus* and *Clostridium difficile.*

### STATE OF THE ART

Seasonal allergies, are widespread and occur only at certain times of the year depending on what triggers the reaction. The most common symptoms mainly affect the mucous membranes lining the nose, inducing allergic rhinitis, or the mucous membranes lining the eyelids and sclera causing allergic conjunctivitis.

Major triggers can be traced to allergens, which in a non-sensitive individual cause no effect, such as pollen or grass. The most common symptoms are itching (especially of the nose, eyes, and palate), nasal drip and obstruction (characterized by a light-colored aqueous secretion), a condition that in children can result in ear infections. Other very common symptoms refer to tearing and eye redness.

Very often individuals with allergic rhinitis also suffer from asthma, characterized by respiratory wheezing, caused by the same allergens that contribute to allergic rhinitis. Depending on the severity of the condition and symptoms, there are several drug treatments that can bring relief in the acute phase of the allergic response. The most common are corticosteroid nasal sprays, which although they do not have strong side effects can lead to epistaxis (nasal bleeding) and a sore nose.

Antihistamine drugs taken orally or through sprays are often used as decongestants, with known side effects including drowsiness, dry mouth, constipation, dizziness, etc. However, the use of spray decongestants are prescribed for a short interval of time without providing any kind of long-term benefit.

Some studies have highlighted the efficacy provided by the use of probiotic bacteria strains in immune support; although opinion is conflicting, the scientific evidence demonstrating their efficacy is not sufficiently established.

In light of the above, there is still a need to find effective, side-effect-free and safe alternative solutions in the prevention and/or treatment of diseases or conditions, including chronic ones, resulting from or related to an alteration of the immune system due to an allergic reaction; in particular, there is a felt need for effective and safe solutions capable of modulating allergic-type inflammatory activity. A commercial composition named Pro-Veflora includes the strains of claim 1 but its use for treating allergic rhinits had not been disclosed.

### SUMMARY OF THE INVENTION

The present invention relates to probiotic compositions comprising:
- L. acidophilus PBS066 (deposit number DSM 24936), L. rhamnosus LRH020 (DSM 25568), B. breve BB077 (LMG P-30157), and B. longum BLG240 (LMG P-29511), for use in the prevention and/or treatment of allergic rhinitis.

### DETAILED DESCRIPTION OF THE INVENTION

The present inventionis related to the probiotic compositions for use as set out in claim 1.

Surprisingly, it has been found that such compositions are effective in modulating allergy-type inflammatory activity, particularly of adults and the elderly and subjects of pediatric age such as children from 4 to 12 years old, and, therefore, are useful compositions in the prevention and/or treatment of allergies, respiratory allergies, preferably seasonal allergies such as hay fever, allergic rhinitis, allergic rhinoconjunctivitis, and allergic asthma, or allergies related to *Staphylococcus aureus* and *Clostridium difficile* in respect of all categories of the population, but particularly in respect of the weaker categories from an immune system point of view, such as of adults and the elderly and subjects of pediatric age such as children from 4 to 12 years old.

In this regard, it has been observed that infections by *Staphylococcus aureus* and *Clostridium difficile* occur more often and with greater recurrence and intensity in allergic subjects. Advantageously, the antimicrobial activity exerted by the compositions of the invention, coupled with the ability to modulate allergic-type inflammatory activity, allows the said compositions to be used against allergic subjects suffering from infections caused by the pathogens *Staphylococcus aureus* and *Clostridium difficile.*

According to the present invention, immune defenses are positively modulated, that is, the activity of the immune system is regulated so that the human body is able to provide an effective immune response against foreign agents, In the present invention, the strains used belong to the species: *Lactobacillus rhamnosus* and *Lactobacillus acidophilus* are selected from *Lactobacillus rhamnosus* LRH020 and *Lactobacillus acidophilus* PBS066 (also known as LA001), and strains belonging to the genus *Bifidobacterium* of the species: *Bifidobacterium longum* and *Bifidobacterium breve* are selected from *Bifidobacterium longum* BLG240 (also known as PBS108) and *Bifidobacterium breve* BB077 (also known as PBS077).

The strain of *Lactobacillus rhamnosus* (*L rhamnosus* or LRH) named "LRH020" was deposited at DSMZ - Deutsche Sammlung von Mikroorganismen und Zellkulturen GmbH under the accession number "DSM 25568" on 17/01/2012 according to the Budapest Treaty. In addition, it was also deposited at BCCM-LMG - Belgian Coordinated Collections of Micro-organisms (BCCM) Laboratorium voor Microbiologie - Bacteriënverzameling (LMG), University Gent on 8/4/2016 under the accession number LMG P-29513.

The strain of *Lactobacillus acidophilus* (*L. acidophilus* or LA) named "PBS066" was deposited at DSMZ - Deutsche Sammlung von Mikroorganismen und Zellkulturen GmbH under the accession number "DSM 24936" on 17/06/2011 according to the Budapest Treaty. In addition, it was also deposited at BCCM-LMG - Belgian Coordinated Collections of Micro-organisms (BCCM) Laboratorium voor Microbiologie - Bacteriënverzameling (LMG), University Gent with the name "LA001" on 8/4/2016 under the accession number LMG P-29512.

The strain of *Bifidobacterium breve* (*B. breve* or BB) named "PBS077" was deposited at DSMZ - Deutsche Sammlung von Mikroorganismen und Zellkulturen GmbH under the accession number "DSM 25173" on 09/14/2011 according to the Budapest Treaty. In addition, it was also deposited at BCCM-LMG - Belgian Coordinated Collections of Micro-organisms (BCCM) Laboratorium voor Microbiologie - Bacteriënverzameling (LMG), University Gent with the name "BB077" on 09/06/2017 under the accession numberLMG P-30157.

The strain of *Bifidobacterium longum* (*B. longum* or BLG) named "PBS108" was deposited at DSMZ - Deutsche Sammlung von Mikroorganismen und Zellkulturen GmbH under the accession number "DSM 25174" on 14/09/2011 according to the Budapest Treaty. In addition, it was also deposited at BCCM-LMG - Belgian Coordinated Collections of Micro-organisms (BCCM) Laboratorium voor Microbiologie - Bacteriënverzameling (LMG), University Gent with the name "BLG240" on 08/04/2016 under the accession number LMG P-29511.

Compositions can be formulated by conventional methods. Preferred forms of administration are solid formulations, e.g., hard capsules, sachets, tablets, sticks, mouth-soluble sticks, tablets, granules, or liquid formulations, e.g., vials with single- or multi-dose dispensing cap, multi-dose dispersions in oil phase, drops, syrups, multiphase emulsions etc. *Lactobacillus rhamnosus* LRH020 strain, may be present in the composition, as percentage by weight with respect of the total weight of the probiotic combination, preferably from 5% to 25%, preferably from 10% to 20%, even more preferably is equal to 15%, *Lactobacillus rhamnosus* LRH020 strain, may be present in each individual unit dose in amounts ranging from 0.5 to 2.5 billion CFU, preferably in amounts ranging from 1 to 2 billion CFU, more preferably it is present in amount of 1 billion CFU. *Lactobacillus acidophilus* strain PBS066, may be present ir the composition, as percentage by weight with respect to the total weight of the probiotic combination, preferably from 5% to 55%, preferably from 15% to 50%, even more preferably is equal to 45% and 21%. *Lactobacillus acidophilus* strain PBS066, may be present in each individual unit dose in amounts ranging from 0.5 to 2.5 billion CFU, preferably in amounts ranging from 1 to 2 billion CFU, more preferably it is present in amount of 1 billion CFU. *Bifidobacterium breve* strain BB077, may be present in the composition, as percentage by weight with respect to the total weight of the probiotic combination, from 5% to 25%, preferably from 10% to 20%, even more preferably is equal to 15%. *Bifidobacterium breve* strain BB077, may be present in each individual unit dose in amounts ranging from 0.5 to 2.5 billion CFU, preferably in amounts ranging from 1 to 2 billion CFU, more preferably it is present in amount of 1 billion CFU. *Bifidobacterium longum* strain BLG240, may be present in the composition, as percentage by weight with respect to the total weight of the probiotic combination, preferably from 30% to 50%, preferably from 35% to 45%, even more preferably is equal to 37% and *42%. Bifidobacterium longum* strain BLG240, may be present in each individual unit dose in amounts ranging from 0.5 to 2.5 billion CFU, preferably in amounts ranging from 1 to 2 billion CFU, more preferably it is present in amount of 1 billion CFU.

The species present in the probiotic combination as reported in Example 3 can be present in a weight ratio of 1.42:1:1:2.85 or 1:1:1:1 when expressed as CFU.

The probiotic compositions of the invention can be administered orally or topically (e.g., nasally or auricularly).

It is the object of the present invention the use of probiotic compositions, which can modulate allergy-type inflammatory activity and enable use in the prevention and/or treatment of allergies, respiratory allergies, preferably seasonal allergies such as hay fever, allergic rhinitis, allergic rhinoconjunctivitis and allergic asthma, or allergies related to *Staphylococcus aureus* and *Clostridium difficile.*

The compositions comprise:
- *L. acidophilus* PBS066 and *L. rhamnosus* LRH020,
- *B. breve* BB077; and *B.longum* BLG240; and these compositions are used to modulate allergy-type inflammatory activity and in the prevention and/or treatment of allergies, respiratory allergies, preferably seasonal allergies such as hay fever, allergic rhinitis, allergic rhinoconjunctivitis, and allergic asthma, or allergies related to *Staphylococcus aureus* and *Clostridium difficile.* The compositions are preferably administered in a dosage range from 1 to 10 billion/CFU/day, preferably from 2 billion/CFU/day to 6 billion/UFC/day, e.g. a dosage of 4 billion/CFU/day corresponding to a dosage of 1 billion/CFU/day for each strain.

Preferred embodiments of the present invention (FRn) are given below:
FR1. A probiotic composition comprising or alternatively consisting of the following strains of bacteria:
   - *L. acidophilus* PBS066 and *L. rhamnosus* LRH020; and
   - *B. breve* BB077 and *B. longum* BLG240, for use ir the prevention and/or treatment of allergies, respiratory allergies, seasonal allergies, such as hay fever, allergic rhinitis, chronic seasonal allergic rhinitis, allergic rhinoconjunctivitis, and allergic asthma, or allergies related to *Staphylococcus aureus* and *Clostridium difficile.*

FR2 The composition for use according to any one of FR1-FR4, wherein said composition is for use in chronic seasonal allergic rhinitis.

FR3 The composition for use according to any one of FR1-FR5, wherein said composition is for use in a method of treatment, preventive or curative, of infections caused by the pathogens *S. aureus* and/or *C. difficile,* preferably in a subpopulation of allergic subjects, as said composition has antimicrobial activity against said pathogens.

FR4 The composition for use according to any one of FR1-FR6, wherein said composition is administered orally or topically.

FR5 The composition according to FR1, wherein said composition is formulated as a food supplement.

FR6 The composition for use according to any one of FR1-FR8, wherein said composition is intended for adults, the elderly, and pediatric subjects, preferably children from 4 to 12 years old. The examples below further illustrate the invention.

### EXAMPLES

### Examples of formulation

### Example 1

A composition has been prepared in the form of a single-dose vial with a dispensing cap:

**Table 1**

| Ingredients | % w/w | Billions CFU/dose |
|---|---|---|
| Solid phase | | |
| *L. rhamnosus LRH20* | 8.87 | 2 |
| *L. acidophilus PBS066* | 13.30 | 2 |
| Maltodextrin | 77.83 | - |
| | 100.0 | 4.0 |
| Liquid phase | | |
| Purified water | 98.61 | - |
| Potassium Sorbate | 0.1 | - |
| Sodium Benzoate | 0.1 | - |
| Aroma | 0.1 | - |
| Acidifier: citric acid | 0.1 | - |
| Vitamin C | 0.96 | - |
| Sweetener | 0.03 | - |
| | 100 | |

### Example 2

A composition has been prepared in stick form:

**Table 2**

| Ingredients | % w/w | Billions CFU/dose |
|---|---|---|
| *L. rhamnosus* LRH020 | 2.33 | 2 |
| *L. acidophilus* PBS066 | 3.50 | 2 |
| *B. breve* BB077 | 1.40 | 2 |
| Vitamin D3 | 0.06 | - |
| Vitamin B6 | 0.05 | - |
| Sorbitol | 24.98 | - |
| FOS | 24.98 | - |
| Maltodextrin | 42.56 | - |
| Silicon dioxide | 0.15 | - |
| | 100.00 | 6.00 |

### Example 3

A composition has been prepared in capsule form:

**Table 3**

| Ingredients | % w/w | Billions CFU/dose |
|---|---|---|
| *L. rhamnosus* LRH020 | 3.90 | 1 |
| *L. acidophilus* PBS066 | 5.85 | 1 |
| *B. breve* BB077 | 2.34 | 1 |
| *B. longum* BLG240 | 11.69 | 1 |
| Maltodextrin | 74.82 | - |
| Magnesium stearate | 0.70 | - |
| Silicon dioxide | 0.70 | - |
| | 100 | 4.00000 |

### Example 4

A composition has been prepared in the form of a single-dose vial with a dispensing cap:

**Table 4**

| INGREDIENTS | % w/w | BILLlON CFU/DOSE |
|---|---|---|
| Solid phase - cap | | |
| *L. acidophilus* PBS066 | 15.22 | 1 |
| *L. rhamnosus* LRH020 | 10.14 | 1 |
| *B. breve* BB077 | 10.14 | 1 |
| *B. longum* BLG240 | 30.43 | 1 |
| Maltodextrin | 34.07 | - |

| Liquid phase | | |
|---|---|---|
| Purified water | 99.1 | - |
| Potassium Sorbate | 0.1 | - |
| Sodium Benzoate | 0.1 | - |
| Aroma | 0.2 | - |
| Acidifier: citric acid | 0.3 | - |
| Sweetener | 0.2 | - |

### EXPERIMENTAL EXAMPLES

The following experimental examples are related to the evaluation of the efficacy of the compositions of the invention.

### Example 2 - Modulation in allergies

Several *in-vitro* experiments were conducted to determine the efficacy of the individual strains reported in Example 1 in modulating allergy-like inflammatory activity.

### CYTOKINE MODULATION IFN - γ, IL - 12, IL - 4 and IL - 10

Allergic disorders are characterized by an inappropriate type 2 (Th-2) immune response against common antigens (such as dust, animal hair, etc.). Interferon gamma (IFN-y), is the main effector of the type 1 (Th-1) inflammatory response, which is known to be crucial during modulation of allergic immunopathologies. Reduction of IL-12 cytokine is related to decreased stimulation of IgE synthesis and allergic-type immune response. At the same time, activation of the allergic-type (Th-2) immune response results in increased production of IL-4 and IL-10 cytokines. These cytokines induce the differentiation of T-naïve cells toward an allergic-type response. The IL-10 cytokine is a potent inhibitor of macrophage activity, negatively modulating the production of pro-inflammatory cytokines needed to rebalance the Th1-Th2 response.

### Materials and methods

Cytokine concentration was measured using peripheral blood mononuclear cells (PBMCs) derived from healthy donors.

Before inoculation, probiotic strains were grown overnight under controlled temperature and humidity in MRS culture medium. Then, aliquots of probiotics were inoculated into the medium containing PBMCs, and depending on the cytokine to be assayed, they were incubated for 24 hours or 5 days under controlled temperature and humidity (IL-12 and IL-10 after 24 hours, and INF- γ and IL-4 after 5 days).

At the end of the incubation time, following centrifugation, the supernatant was recovered and stored at - 20°C for immunoassay.

The assay was performed by using a commercial sandwich ELISA kit following the instructions given. The experiments conducted were compared with two controls one positive (system perturbed with LPS, in the absence of probiotics) and one negative control (basal condition with no perturbation).

### Results

Probiotic strain BLG240 is able to increase the concentration of interferon-y, the main mediator in polarizing cells toward a type 1 (Th-1) immune response as shown in Table 5.

**Table 5**

| | *IFN-γ* (*pg*/*mL*) |
|---|---|
| *CTR-* | 30.0 |
| *CTR+* | 535.0 |
| *BLg - BLG240* | 330.0 |

In contrast, on the other hand, the two single strains BLG240 and BB077 were able to reduce the concentration of IL-12p70 cytokine, which is responsible for the increased allergic-type inflammatory response (Table 6).

**Table 6**

| | *IL-12p70 (pg*/*mL)* |
|---|---|
| *CTR-* | 30.0 |
| *CTR+* | 130.0 |
| *BLg - BLG240* | 35.0 |
| *BB - BB077* | 70.0 |

From Tables 7 and 8, it can be seen that the single strains, BLG240 for IL-4 and IL-10 while BB077 only for IL-10, belonging to the probiotic composition shown in Example 3, are able to positively modulate the immune response, re-balancing it toward the Th-1 type response, by reducing compared to the positive control the concentration of the above-mentioned cytokines.

**Table 7**

| | *IL-4* (*pg*/*mL*) |
|---|---|
| *CTR-* | *15.0* |
| *CTR+* | *48.0* |
| *BLG - BLG240* | *35.0* |

**Table 8**

| | *IL-10 (pg*/*mL)* |
|---|---|
| *CTR-* | 34.0 |
| *CTR+* | 360.0 |
| *BLg - BLG240* | 230.0 |
| *BB - BB077* | 325.0 |

### MODULATION OF TUMOR NECROSIS FACTOR a

The probiotic strains reported in Example 3 in addition to being able to positively modulate the allergy-like response, they also demonstrated a remarkable ability to positively modulate the concentration of TNF-a, the first pro-inflammatory cytokine recruited during the type 1 immune response.

### Materials and methods

The viability of cell cultures (BALB/c3T3, clone A31) treated with SDS (sodium dodecyl sulfate, irritant/proinflammatory agent - exposure time: 24 hours and 5 days) in the presence and absence of probiotics of the invention was evaluated.

The results are compared with those obtained for positive control cells treated exclusively with SDS (CTR+) and negative control cells without any treatment (CTR-).

### Results

All the individual strains within the probiotic composition reported in Example 3 have been shown to significantly reduce the concentration of the pro-inflammatory cytokine in the acute phase. This result is in line with cellular physiology considering that TNF-a is one of the cytokines involved in the acute phase of the inflammatory response (Table 9).

**Table 9**

| *TNF-a* | *24h* | *5g* |
|---|---|---|
| *CTR-* | 139.7 | 932.3 |
| *CTR+* | 406.2 | 2706.9 |
| *LRh - LRH020* | 158.4 | 857.1 |
| *BB - BB077* | 154.2 | 862.1 |

### MODULATION OF CYTOKINE IL-4

Interleukin-4 is known to be an anti-inflammatory cytokine, and its presence tends to increase during an inflammatory response.

### Materials and methods

The viability of cell cultures (BALB/c3T3, clone A31) treated with SDS (sodium dodecyl sulfate, irritant/proinflammatory agen t- exposure time: 24 hours and 5 days) in the presence and absence of probiotics of the invention was evaluated.

The results are compared with those obtained for positive control cells treated exclusively with SDS (CTR+) and negative control cells without any treatment (CTR-).

### Results

All of the individual strains within the probiotic mixture reported in Example 3 have been shown to significantly increase the concentration of the anti-inflammatory cytokine in both an acute-24-hour condition (Table 10). In the 5-day chronic condition, for example, both strain LA - PBS066 and strain LRh -LRH020 increased the concentration of the anti-inflammatory cytokine IL-4 more than CTR+.

**Table 10**

| *IL-4* | *24h* |
|---|---|
| *CTR-* | *516.5* |
| *CTR+* | *584.2* |
| *LA - PBS066* | *631.4* |
| *LRh - LRH020* | *752.6* |
| *BLg - BLG240* | *646.9* |
| *BB - BB077* | *617.0* |

### MODULATION OF SENSITIZING MARKERS

Allergens are able to induce phenotypic alterations on dendritic cells, increase both the production of cytokines involved in the allergic response and the number of receptors (CD54 or CD86) on the cell surface responsible for activating T cells toward an allergic-type response.

### Materials and methods

The *in-vitro* ability of probiotics to modulate the number of CD54 and CD86 receptors present on the surface of THP1 monocytes was assessed by treatment of the cell line with a sensitizing agent (2,4-dinitrochlorobenzene-DNCB). Then, by cytofluorometer, the fluorescence index was measured relative to the individual strains compared with the negative control (system not perturbed by DNCB) and positive control (system perturbed, but in the absence of probiotics).

### Results

The probiotic composition reported in Example 3, has been shown to significantly inhibit the action of sensitizing markers through positive modulation of CD54 and CD86 receptor expression (Tables 11 and 12).

**Table 11**

| *SAMPE* | *RFI %* |
|---|---|
| *CTR-* | *100.00* |
| *CTR+* | *272.33* |
| *LRh - LRH020* | *179.00* |
| *BB - BB077* | *190.67* |
| *LA* - *PBS066* | *174.33* |
| *BLg - BLG240* | *183.00* |
| *Probiotic composition example 3* | *209.00* |

**Table 12**

| *SAMPLE* | *RFI* % |
|---|---|
| *CTR-* | 100.00 |
| *CTR+* | 197.00 |
| *LRh - LRH020* | 61.00 |
| *BB - BB077* | 143.67 |
| *LA - PBS066* | 142.67 |
| *BLg - BLG240* | 102.33 |
| *Probiotic composition example 3* | 164.33 |

### Antimicrobial activity

It has been observed that infections by *S. aureus* and *C. difficile* occur more often in allergic individuals. Single strains LRH020 and PBS066 belonging to the probiotic composition reported in Example 3 have been shown *in-vitro* to have strong antimicrobial activity against *S. aureus.*

In contrast, the single strains LRH020 and PBS066 belonging to the probiotic composition reported in Example 3 have been shown *in-vitro* to have strong antimicrobial activity against *C. difficile.*

The aforementioned single strains demonstrated remarkable capabilities in both a "preventive" model - plate growth of the probiotic and subsequent *overlay* of the pathogenic strain--and in the "treatment" model - plate growth of the pathogen followed by addition of the culture medium of the post-biotic-enriched probiotic strains released during fermentation (Table 13 and Table 14).

**Table 13**

| Probiotic strain | Prevention model | Treatment model |
|---|---|---|
| | *S. aureus* | *S. aureus* |
| LRh-LRH020 | ** | ** |
| LA -PBS066 | ** | ** |

**Table 14**

| Probiotic strain | Prevention model | Treatment model |
|---|---|---|
| | *C. difficult* | *C. difficult* |
| LRh-LRH020 | ** | ** |
| LA -PBS066 | * | ** |

| | | |
|---|---|---|
| Legend: ** strong inhibition; * moderate inhibition. | | |

**Clinical study protocol:** Randomized, double-blind placebo-controlled, parallel-group clinical trial on the clinical efficacy of a probiotic product in improving symptoms of perennial and seasonal allergic rhinitis.

### Objectives of the study

The main objective of the study is to evaluate the clinical efficacy of a food product, containing a mixture of probiotic strains (*L. acidophilus* PBS066, *L. rhamnosus* LRH020, *B. breve* BB077, and *B*. *longum* BLG240), on allergic rhinitis-related symptoms on subjects with symptoms of seasonal or perennial allergic rhinitis, to establish its effect of improving the immune response. For this reason, evaluations will be made with respect to improvement of typical rhinitis symptoms, improvement of quality of life, and modulation of immunological parameters related to the inflammatory state.

### Trial design

A randomized double-blind, placebo-controlled, parallel-group clinical trial will be conducted on a total of 60 subjects with symptoms of allergic rhinitis, aged 18 to 60 years. Volunteers will be divided into 2 parallel groups of 30 subjects who will be given, according to randomization list, the study product containing probiotics and a placebo product for 8 weeks. This will be followed by a follow-up period of another 4 weeks.

Assessments regarding health status and treatment efficacy will be made at recruitment, at the beginning of the study, at mid-treatment, at the end of treatment, and after the follow-up period. The study was planned with consideration of a treatment period that was adequate to be able to ensure intestinal colonization of probiotic strains and subsequent improvement of symptoms.

### Inclusion criteria

Healthy subjects of either sex aged 18 to 60 years:
- who have symptoms of seasonal or perennial allergic rhinitis according to assessment questionnaire (in the case of seasonal rhinitis to be enrolled before the period and during pre-seasonal pollen period)
- who have the willingness and ability to participate in the study
- who have a willingness not to vary their normal daily routine (lifestyle, physical activity, etc.) throughout the duration of the study

- who have a willingness not to vary their normal diet throughout the duration of the study
- who have a willingness to use only the product to be tested throughout the duration of the study
- who have a willingness not to use similar products throughout the duration of the study
- who have a willingness not to use products that may interfere with the product to be tested
- who have not recently participated in similar studies
- who have signed the informed consent and are aware of the study procedures.

### Exclusion criteria

Subjects with the following characteristics:
- who do not fit the inclusion criteria
- with suspected or established sensitivity to one or more components of the product
- with an established history of chronic disease (congenital cardiovascular disease, liver and kidney disease, or immunodeficiency)
- undergoing drug and/or antibiotic treatment (in place)
- who cannot discontinue medications that may affect rhinitic symptoms during the study period
- who have other ongoing concomitant diseases (immune, infectious, respiratory, or gastrointestinal)
- with uncontrolled asthma
- undergoing systemic steroid treatment within 2 weeks from the start of the study
- with gastritis and undergoing proton pump or antiH2 treatment
- undergoing recent (less than 3 months) immunosuppressant therapy
- with ongoing serious diseases
- who abuse drugs and/or alcohol
- who are deemed by the investigator to be ineligible for participation for any reason
- who are unable to communicate or cooperate with the experimenter due to language problems, mental retardation, or impaired brain function.

### Interventions

The study will be conducted on two parallel groups of volunteers, one group given a probiotic product (treatment) and one group given a product containing only excipients (placebo).

After enrollment, the two groups will be given one of the two products, according to randomization list.

Evaluation of the effects of the tested product will be carried out after 8 weeks of treatment and after a follow-up period of another 4 weeks (total period=12 weeks).

The products to be administered to the volunteers during the study will be the active product, object of the study, containing a composition of probiotic strains (*L. acidophilus* PBS066, *L. rhamnosus* LRH020, *B. breve* BB077 and *B. longum* BLG240) added with excipients normally used in the formulation of food supplements, and a placebo product containing excipients alone, according to formulas below:

### Product from Example 3

**Table 15**

| ***Ingredients*** | ***mg*/*cps*** |
|---|---|
| *L. acidophilus* PBS066 | 25 |
| *L. rhamnosus* LRH020 | 16.667 |
| *B. breve* BB077 | 16.667 |
| *B*. *longum* BLG240 | 50 |
| Maltodextrin | 167.566 |
| Cornstarch | 72 |
| Magnesium stearate Magnesium stearate | 3.55 |
| Silicon dioxide | 3.55 |
| Capsule size 0 DR | 95 |
| **Total** | 450 |

### Placebo Product

**Table 16**

| ***Ingredients*** | ***mg*/*cps*** |
|---|---|
| Maltodextrin | 275.9 |
| Cornstarch | 72 |
| Magnesium stearate | 3.55 |
| Silicon dioxide | 3.55 |
| Capsule size 0 DR | 95 |
| **Total** | 450 |

It is recommended for both the probiotic product and placebo to use 1 capsule per day.

### Outcomes

Primary outcome of the study will be the demonstration of a statistically significant difference in allergic rhinitis symptoms before and after treatment. To determine the decrease in the persistence and severity of symptoms (nasal itching, sneezing, rhinorrhea, nasal obstruction), an assessment will be made according to the *Total Nasal Symptom Score* (TNSS) questionnaire, which will be completed at each visit with the help of the referring physician. The questionnaire will be provided in Italian. For each question, the significant difference in TNSS score between the two groups (treated versus placebo) before and after treatment will be considered as improvement.

Secondary outcomes will be the assessment of improvement in quality of life using the *Mini Rhinoconjunctivitis Quality of Life Questionnarie* (MiniRQLQ) and an improvement in inflammatory and fecal markers, pre- and post-treatment compared with placebo. For quality of life assessment, the increase in the total score of the questionnaire will be taken into account: the lower the score, the better the quality of life will be considered. For secondary outcomes, in addition to completing the questionnaire on the persistence of allergic rhinitis symptoms and quality of life, blood samples for analysis of serological biomarkers and fecal samples will be collected during visits.

Assessment of reduction in inflammatory status will be made by analysis of blood inflammatory markers: eosinophil count on blood and eosinophil cationic protein.

### Timeline

The study includes an initial visit with the referring physician during which recruitment will take place, followed by four additional visits:
- Recruitment visit and medical history 2 weeks before the start of the study (T-1)
- Enrollment visit, randomization, and start of the study (T0)
- Checkup visit at 4 weeks (T1)
- Objective observation visit at 8 weeks (T2)
- Follow-up visit at 12 weeks (T3)

The procedures followed during the visits are described below.

### Initial recruitment visit (T-1)

During the initial visit, the referring physician will verify that the subject is eligible to participate in the study. The following procedures will also be carried out:
- Visit, medical history, recruitment and detailed explanation of the study protocol to volunteers
- Master data collection
- Signature of informed consent and privacy policy
- Completion of evaluation questionnaires

### Enrollment visit and start of the study (T0)

During the enrollment visit, the referring physician, based on the questionnaires completed during the T-1 visit, will report that the subject is eligible to participate in the study. The following procedures will also be carried out:
- Completion of evaluation questionnaires
- Product/placebo delivery according to randomization list
- Collection of blood samples to be used for analysis of inflammatory parameters
- Collection of fecal samples that will be stored for gut microbiome analysis. After the visit, the subject will proceed with taking the product.

### Interim visit (T1=4 weeks)

After 4 weeks from the start of taking the product, a third visit will be made at which the referring physician will verify that the subject is still eligible to participate in the study and that no adverse events have been observed. The following procedures will also be carried out:
- Objective examination and observation
- Product tolerability verification and compliance
- Completion of evaluation questionnaires

### Interim visit (T2=8 weeks)

After 8 weeks from the start of taking the product, a fourth visit will be made at which the referring physician will verify that no adverse events have occurred. The following procedures will also be carried out:
- Objective examination and observation
- Product tolerability and compliance verification
- Completion of evaluation questionnaires
- Collection of blood samples to be used for analysis of inflammatory parameters
- Collection of fecal samples that will be stored for gut microbiome analysis

### Final visit (T3=12 weeks)

After 4 weeks from the end of treatment, a final visit will be made at which the referring physician will check for adverse events. The following procedures will also be carried out:
- Objective examination and observation
- Product tolerability and compliance verification
- Completion of evaluation questionnaires
- Collection of salivary samples to be used for analysis of inflammatory parameters
- Collection of fecal samples that will be stored for gut microbiome analysis
- Return of any remaining product

### Sample size

The sample size was estimated based on the effect on the study outcome, i.e., the percentage of patients estimated to have an improvement in typical symptoms of allergic rhinitis compared with the initial condition. Based on data in the relevant scientific literature, the study was designed to show sufficient power (80%) to detect a difference between groups after treatment at a significance level of 0.05.

The expected benefit is an improvement in symptoms (including nasal itching, sneezing, rhinorrhea, nasal obstruction) compared with the initial condition in 60 percent of subjects for the placebo product and 85 percent of subjects for the active product.

### DATA COLLECTION MANAGEMENT AND ANALYSIS

For the determination of the primary outcome, questionnaires for the assessment of allergic rhinitis symptoms will be administered at each visit and then kept by the staff in charge in dedicated binders until the end of the study for appropriate evaluation. For data interpretation, the investigator will use the conceptual scheme of the TNSS diagnostic tool.

The same procedure as for the symptom assessment questionnaires will be followed for the quality of life questionnaires.

Regarding inflammatory markers, blood samples will be taken at the T0, T2 and T3 visit by trained personnel and then sent to the reference analysis laboratory according to standardized procedures. The analysis laboratory will proceed to perform related tests: eosinophil count on blood and eosinophil cationic protein. The data obtained will be processed and the results sent to the Promoter who will proceed with statistical analysis of the same.

Regarding the gut microbiome analysis, fecal samples will be collected at T0, T2 and T3. The samples will be stored and analyzed later by *Next Generation Sequencing* (NGS) to study the evolution of gut microbiome biodiversity during the study period (treatment and follow-up).

Treatment efficacy will be evaluated based on all those subjects who complete the study without protocol variation. Subjects who:
- will not show up for checkup visits after 8 weeks of product use or after the 4-week follow-up
- will not use the product properly during the study period

### Statistical methods

### Descriptive analysis

Demographic variables (age, sex, etc.) will be reported and the data obtained will be summarized using frequency distributions (number and percentage).

The following value will be calculated for all continuous variables:
- the average value
- the minimum value
- the maximum value
- the standard deviation
- the individual change / the individual percentage change
- the average change / the average percentage change

### Statistical analysis

Clinical data will be analyzed by ANOVA test followed by Tukey-Kramer test (intra-group analysis); intergroup statistical analysis will be performed on data changes from T0 by Student's bilateral t-test for unpaired data.

The following comparison will be made for each parameter:
- Intra-group comparison: comparison of all experimental times vs T0
- Intergroup comparison: comparison of the two study groups at all experimental times.

The statistical software used for statistical analysis is: NCSS 10 statistical software (NCSS, LLC. Kaysville, Utah, USA) running on Windows Server 2008 R2 Standard (Microsoft, USA).

### MONITORING

Because this was a double-blind study, it was not deemed necessary to form a data monitoring committee.

### Improvement of symptoms of perennial and seasonal allergic rhinitis - clinical study

### Materials and methods

A randomized double-blind placebo-controlled parallel-group clinical trial was conducted on 60 subjects with symptoms of allergic rhinitis, aged 18 to 60 years. The volunteers were evenly divided into two groups of 30 subjects who were administered, according to randomization list, either the probiotic composition given in Example 3/Table 3 (dosage of 4 Billion CFU/dose) or placebo (Table 17) for 8 weeks followed by a follow-up period of another 4 weeks. Visits were scheduled as follows:
- Recruitment visit and medical history 2 weeks before the start of the study (T-1)
- Enrollment visit, randomization, and start of the study (T0)
- Checkup visit at 4 weeks (T1)
- Objective observation visit at 8 weeks (T2)
- Follow-up visit at 12 weeks (T3)

**Table 17**

| **Ingredients** | **mg/cps** |
|---|---|
| Maltodextrin | 275.9 |
| Cornstarch | 72 |
| Magnesium stearate | 3.55 |
| Silicon dioxide | 3.55 |
| Capsule size 0 DR | 95 |
| **Total** | **450** |

During the initial visit (T-1), the referring physician verified that the subject was eligible to participate in the study. The following procedures were also performed:
- Visit, medical history, recruitment and detailed explanation of the study protocol to volunteers
- Master data collection
- Signature of informed consent and privacy policy
- Completion of evaluation questionnaires

During the enrollment visit (T0), the referring physician, based on the questionnaires completed during the T-1 visit, reported the subject's eligibility is fit to participate in the study. The following procedures were also carried out:
- Completion of evaluation questionnaires
- Product/placebo delivery according to randomization list
- Collection of blood samples to be used for analysis of inflammatory parameters
- Collection of fecal samples that will be stored for gut microbiome analysis

After the visit, the subject started taking the product.

After 4 weeks from the start of taking the product, a third visit was made in which the referring physician verified that the subject was still eligible to participate in the study and that no adverse events had been observed. The following procedures were also performed:
- Objective examination and observation
- Product tolerability and compliance verification
- Completion of evaluation questionnaires

After 8 weeks from the start of taking the product, a fourth visit was made at which the referring physician verified that no adverse events had been observed. The following procedures were also performed:
- Objective examination and observation
- Product tolerability and compliance verification
- Completion of evaluation questionnaires
- Collection of blood samples to be used for analysis of inflammatory parameters
- Collection of fecal samples that will be stored for gut microbiome analysis

After 4 weeks from the end of treatment, the last visit was made at which the referring physician verified that no adverse events had been observed. The following procedures were also performed:
- Objective examination and observation
- Product tolerability and compliance verification
- Completion of evaluation questionnaires
- Collection of salivary samples to be used for analysis of inflammatory parameters
- Collection of fecal samples that will be stored for gut microbiome analysis
- Return of any remaining product

### Results

### Analysis of allergy markers

Evaluation of reduction in inflammatory and allergic status was assessed by analysis of blood markers such as eosinophil count on blood and eosinophil cationic protein.

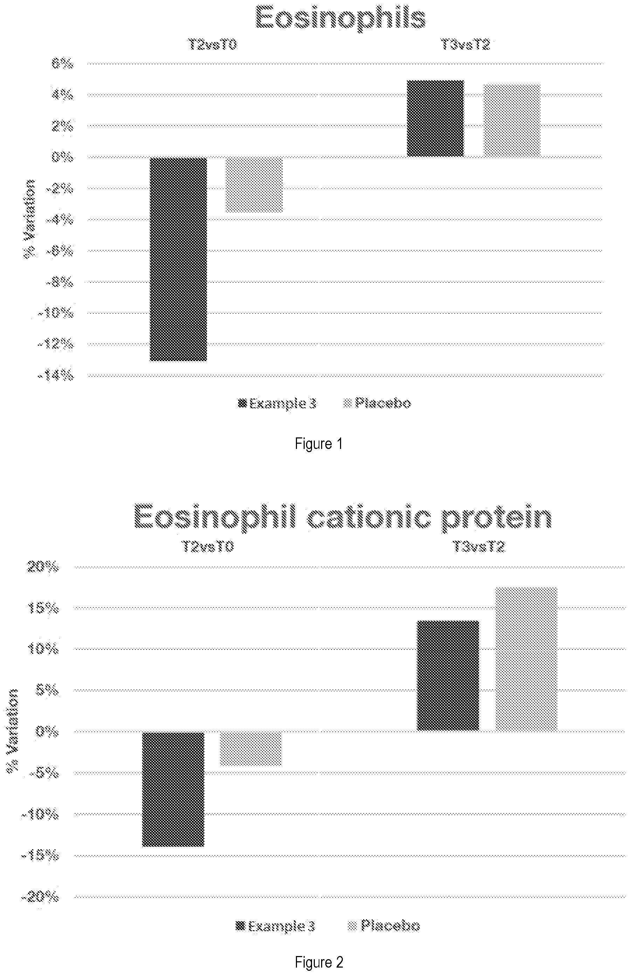

Eosinophilic inflammation of the nasal mucosa is a typical feature of allergic rhinitis, often related and comparable to systemic inflammation. In patients treated with the active product, a strong decrease in the concentration of eosinophils was observed during administration, as opposed to the placebo group, while in the follow-up this difference decreased given the resolution of symptoms and the end of the allergic season (Figure 1).

**Table 18**

| Eosinophils | | |
|---|---|---|
| | T2vsT0 | T3vsT2 |
| Probiotic group | -13% | 5% |
| Placebo | -4% | 5% |

This trend was also confirmed by the observed trend in eosinophil-released metabolites responsible for nasal epithelial damage and allergic hyperreactivity, such as eosinophil cationic protein (ECP). Specifically, the group treated with the product in Example 3 showed a reduction in ECP concentration during the two months of treatment, compared with placebo, an effect that moderated in follow-up given the resolution of symptoms and the end of the allergic season (Figure 2).

**Table 19**

| Eosinophil cationic protein | | |
|---|---|---|
| | T2vsT0 | T3vsT2 |
| Probiotic group | -14% | 13% |
| Placebo | -4% | 17% |

### Symptoms questionnaires

To assess the decrease in the persistence and severity of symptoms (nasal itching, sneezing, rhinorrhea, nasal obstruction), an assessment was made according to the TNSS questionnaire, which evaluates the symptoms of allergic rhinitis, wherein lower scores correspond to milder symptoms. In subjects in the active group (example 3) there is a significant reduction in score for all times tested (T1, T2 and T3) compared with the initial score at T0 (p < 0.05).

The MiniRQLQ questionnaire was used to assess the impact of allergic rhinitis on quality of life: a reduction in score corresponds to a better perception of quality of life.

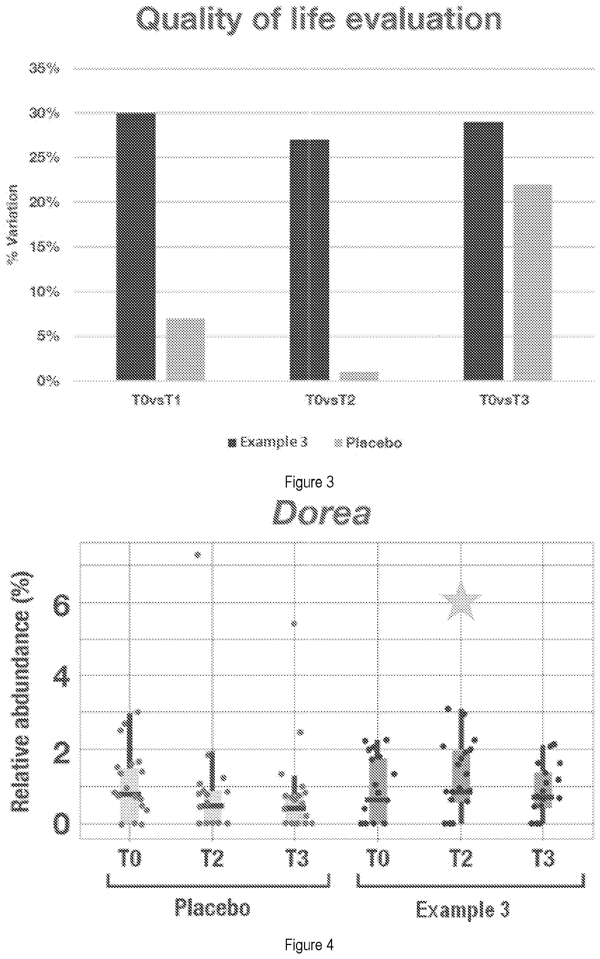

In both groups there is a trend of improvement in quality of life, but only in the active group is there a significant difference at T3 compared to T0. (Figure 3)

**Table 20**

| | Improvement of quality of life | | |
|---|---|---|---|
| | QOL | | |
| | T0vsT1 | T0vsT2 | T0vsT3 |
| Placebo | 7% | 1% | 22% |
| Probiotic group | 30% | 27% | 29% |

### Gut microbiota analysis

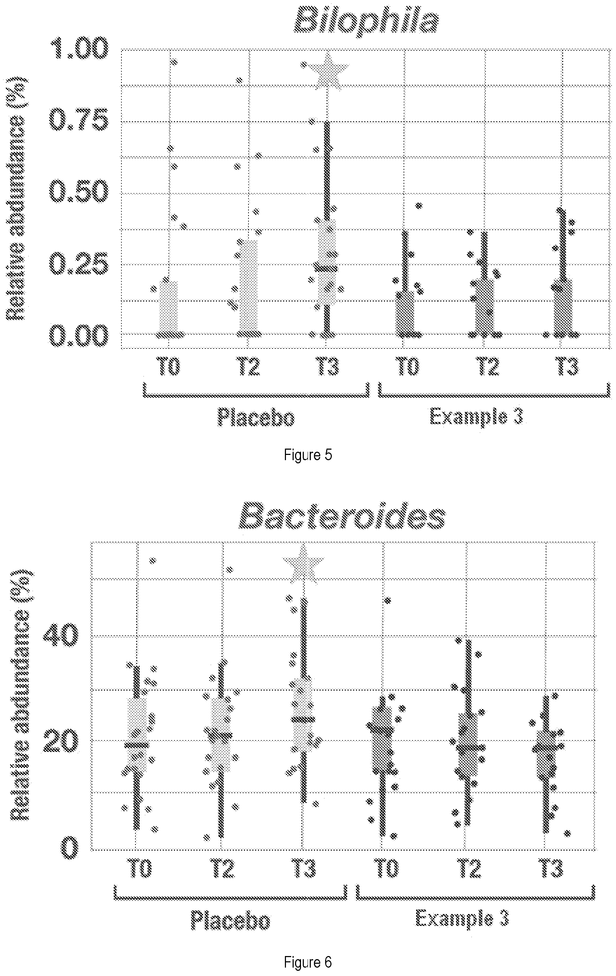

Changes in the fecal microbiota profile were determined by 16S rRNA gene sequencing. The sequenced samples show a richness and phylogenetic diversity of taxa present in the intestinal ecosystem that remains constant during the three time points. From the perspective of genera of medical interest in the occurrence of allergic diseases, it was observed that:
- In the active group, an increase in *Dorea* was observed during the two months of treatment (T2), unlike what was shown in the placebo group (Figure 4). This genus is reported in the literature to be inversely associated with the gut microbiota profile of allergic and atopic subjects. In addition, an increase in the genus *Fusicatenibacter,* known to exert anti-inflammatory activity, was observed at T3.
- In the placebo group, in contrast to the active, there is evidence of an increase in *Bilophila* (Figure 5), *Bacteroides* (Figure 6) and *Ruminococcus* during the treatment period (T2) that continues in the follow-up (T3); the alteration of these genera correlates with a pro-inflammatory condition and the onset of allergic phenomena.

In addition, no significant increases in the administered genera, *Lactobacilli* and *Bifidobacteria,* were observed, indicating an indirect modulation on the subjects' gut microbiota without altering the initial biodiversity.

In conclusion, the probiotic composition of Example 3 showed a clear clinical improvement in the extent and control of symptoms as well as in the quality of life of subjects with chronic seasonal allergic rhinitis, compared with placebo treatment. This effect was also associated with a modulation of the gut microbiota, promoted by an increase in the concentration of genera related to anti-inflammatory and anti-allergic activity. It is interesting to note that the positive trend is relative to the treatment period, supporting the actual benefit exerted by the probiotic treatment during intake. This effect, on the other hand, goes away during the follow-up period and aligns with the placebo trend, given the resolution of symptoms due to the end of the typically allergic season.

## Claims

1. A probiotic composition comprising or alternatively consisting of the following strains of bacteria:
- *L. acidophilus* PBS066, having deposit number DSM 24936, and *L. rhamnosus* LRH020, having deposit number DSM 25568; and
- *B*. *breve* BB077, having deposit number LMG P-30157, and *B*. *longum* BLG240, having deposit number LMG P-29511, wherein said composition is for use in the prevention and/or treatment of allergic rhinitis.

2. The composition for use according to claim 1, wherein said composition is for use in the prevention and/or treatment of chronic seasonal allergic rhinitis.

3. The composition for use according to any one of claims 1-2, wherein said composition is for use in the control of symptoms of chronic seasonal allergic rhinitis.

4. The composition for use according to any one of claims 1-3, wherein said composition is for use in a method of treatment, preventive or curative, of infections caused by the pathogens *S. aureus* and/or *C. difficile,* preferably in a subpopulation of allergic subjects, said composition having antimicrobial activity against said pathogens.

5. The composition for use according to any one of claims 1-4, wherein said composition is administered orally or topically.

6. The composition for use according to claim 1, wherein said composition is formulated as a food supplement.

7. The composition for use according to any one of claims 1-6, wherein said composition is intended for adults, the elderly, and in pediatric subjects, preferably children from 4 to 12 years old.

## Patentansprüche

1. Probiotische Zusammensetzung, die die folgenden Bakterienstämme umfasst oder alternativ daraus besteht:
- *L. acidophilus* PBS066, mit der Hinterlegungsnummer DSM 24936, und *L. rhamnosus* LRH020, mit der Hinterlegungsnummer DSM 25568; und
- *B. breve* BB077, mit der Hinterlegungsnummer LMG P-30157, und *B. longum* BLG240, mit der Hinterlegungsnummer LMG P-29511, wobei die Zusammensetzung zur Verwendung bei der Prävention und/oder Behandlung von allergischer Rhinitis bestimmt ist.

2. Zusammensetzung zur Verwendung nach Anspruch 1, wobei die Zusammensetzung zur Verwendung bei der Prävention und/oder Behandlung von chronischer saisonaler allergischer Rhinitis bestimmt ist.

3. Zusammensetzung zur Verwendung nach einem der Ansprüche 1-2, wobei die Zusammensetzung zur Verwendung bei der Linderung der Symptome von chronischer saisonaler allergischer Rhinitis bestimmt ist.

4. Zusammensetzung zur Verwendung nach einem der Ansprüche 1-3, wobei die Zusammensetzung zur Verwendung in einem Verfahren zur präventiven oder kurativen Behandlung von Infektionen bestimmt ist, die durch die Krankheitserreger *S. aureus* und/oder *C. difficile,* vorzugsweise bei einer Untergruppe allergischer Subjekte, verursacht werden, wobei die Zusammensetzung eine antimikrobielle Wirkung gegen die Krankheitserreger aufweist.

5. Zusammensetzung zur Verwendung nach einem der Ansprüche 1-4, wobei die Zusammensetzung oral oder topisch verabreicht wird.

6. Zusammensetzung zur Verwendung nach Anspruch 1, wobei die Zusammensetzung als Nahrungsergänzungsmittel formuliert ist.

7. Zusammensetzung zur Verwendung nach einem der Ansprüche 1-6, wobei die Zusammensetzung für Erwachsene, ältere Menschen und pädiatrische Subjekte, vorzugsweise Kinder im Alter von 4 bis 12 Jahren, bestimmt ist.

## Revendications

1. Composition probiotique, comprenant ou consistant alternativement en les souches de bactéries suivantes :
- *L. acidophilus* PBS066, portant le numéro de dépôt DSM 24936, et *L. rhamnosus* LRH020, portant le numéro de dépôt DSM 25568 ; et
- *B. breve* BB077, portant le numéro de dépôt LMG P-30157, et *B*. *longum* BLG240, portant le numéro de dépôt LMG P-29511, dans laquelle ladite composition est destinée à être utilisée dans la prévention et/ou le traitement de la rhinite allergique.

2. Composition à utiliser selon la revendication 1, dans laquelle ladite composition est destinée à être utilisée dans la prévention et/ou le traitement de la rhinite allergique saisonnière chronique.

3. Composition à utiliser selon l'une quelconque des revendications 1-2, dans laquelle ladite composition est destinée à être utilisée dans le contrôle des symptômes de la rhinite allergique saisonnière chronique.

4. Composition à utiliser selon l'une quelconque des revendications 1-3, dans laquelle ladite composition est destinée à être utilisée dans une méthode de traitement, préventif ou curatif, d'infections causées par les agents pathogènes *S. aureus* et/ou *C. difficile,* de préférence dans une sous-population de sujets allergiques, ladite composition ayant une activité antimicrobienne contre lesdits agents pathogènes.

5. Composition à utiliser selon l'une quelconque des revendications 1-4, dans laquelle ladite composition est administrée par voie orale ou topique.

6. Composition à utiliser selon la revendication 1, dans laquelle ladite composition est formulée comme complément alimentaire.

7. Composition à utiliser selon l'une quelconque des revendications 1-6, dans laquelle ladite composition est destinée aux adultes, aux personnes âgées et aux sujets pédiatriques, de préférence les enfants de 4 à 12 ans.
